# EUROPEAN PATENT APPLICATION

(11) **EP 4 711 002 A1**
(43) Date of publication of application: **18.03.2026**
(21) Application number: 25199654.2
(22) Date of filing: 02.09.2025
(51) Int. Cl.: A61N 5/10, A61B 6/00, H02K 21/00

(54) **DIRECT-DRIVE SYSTEM FOR GANTRY ROTATION**

(30) Priority: 12.09.2024 US 202418882788
(71) Applicant: Varian Medical Systems, Inc., Palo Alto, CA 94304 (US)
(72) Inventor: GAUDIO, Stephen, San Diego, 92119 (US)
(74) Representative: Mathisen & Macara LLP

(57) **Abstract**

A radiation treatment system 100, 300 includes a base stand 200 fixed to or resting on a support surface external to the radiation treatment system, a rotatable gantry 110 with a treatment-delivering radiation source mounted thereon, wherein the rotatable gantry is rotatably coupled to the base stand via a bearing 205, and an electric motor for rotating the rotatable gantry and a portion of the bearing fixed to the gantry. The electric motor includes a first assembly 510 that is fixed to the base stand; and a second assembly 520 that is fixed to the gantry and is separated from the first assembly by an air gap 501, wherein magnetic flux across the air gap causes the electric motor to rotate the rotatable gantry and the portion of the bearing fixed to the rotatable gantry.

## Description

### BACKGROUND

Unless otherwise indicated herein, the approaches described in this section are not prior art to the claims in this application and are not admitted to be prior art by inclusion in this section.

Radiation therapy (also called radiotherapy) is a cancer treatment that employs high doses of ionizing radiation, such as X-rays or high-energy electrons, protons, or other heavy charged particles, to kill cancer cells. Generally, radiation therapy is a localized treatment for a specific target tissue, such as a cancerous tumor. Ideally, radiation therapy is performed on a planning target volume (i.e., the target tissue) that spares the surrounding normal tissue from receiving doses above specified tolerances, thereby minimizing risk of damage to healthy tissue. For example, to accurately supply a planned radiation dose, the spatial distribution of delivered radiation dose within the patient must closely match the spatial distribution of the planned radiation dose. So that the planned radiation dose is correctly supplied to the planning target volume during radiation therapy, the patient should be correctly positioned relative to the radiation source that provides the radiation therapy. In addition, precisely controlling the position of the radiation source relative to the patient is a significant factor in accurately targeting tissue in the patient. In light of the above, drive systems that enable precise and repeatable rotational positioning of a radiation source about a patient are commonly employed in radiation therapy systems.

### SUMMARY

According to one aspect of the invention, there is provided a radiation treatment system as defined in claim 1. Optional features are specified in the dependent claims. According to this aspect of the invention, the electric motor may be configured to generate the magnetic flux across the air gap to rotate the rotatable gantry and the portion of the bearing fixed to the rotatable gantry.

According to various embodiments, a radiation therapy system may include a drive system that provides accurate and repeatable rotational positioning of a radiation source during radiation therapy. The drive system described herein in some embodiments includes a direct-drive electrical motor with a first assembly that is fixed to the base stand and a second assembly that is fixed to a rotatable gantry on which a treatment-delivering X-ray source is mounted. Unlike conventional drive systems for rotating a gantry of a radiation therapy system, the drive system described herein in some embodiments has no interposing mechanical drivetrain between the first assembly fixed to the base stand and the second assembly fixed to the rotatable gantry. Instead, the first assembly is separated from the second assembly by an air gap, and magnetic flux across the air gap causes the electric motor to rotate the rotatable gantry. In some embodiments, the electric motor has a configuration of magnets and coils capable of providing high torque from a small volume.

According to some embodiments, a radiation treatment system includes: a base stand fixed to or resting on a support surface external to the radiation treatment system; a rotatable gantry with a treatment-delivering radiation source mounted thereon, wherein the rotatable gantry is rotatably coupled to the base stand via a bearing; and an electric motor for rotating the rotatable gantry and a portion of the bearing fixed to the gantry. The electric motor includes: a first assembly that is fixed to the base stand; and a second assembly that is fixed to the gantry and is separated from the first assembly by an air gap, wherein magnetic flux across the air gap causes the electric motor to rotate the rotatable gantry and the portion of the bearing fixed to the rotatable gantry.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

The foregoing and other features of the present disclosure will become more fully apparent from the following description and appended claims, taken in conjunction with the accompanying drawings. These drawings depict only several embodiments in accordance with the disclosure and are, therefore, not to be considered limiting of its scope. The disclosure will be described with additional specificity and detail through use of the accompanying drawings.
FIG. 1 is a perspective view of a radiation therapy system that can beneficially implement various embodiments.
FIG. 2 schematically illustrates a side view of the radiation therapy system of FIG. 1, according to various embodiments.
FIG. 3 is a perspective view of an RT system, according to various embodiments.
FIG. 4 schematically illustrates a base stand and a gantry of the RT system of FIG. 3, according to various embodiments.
FIG. 5 schematically illustrates a more detailed view of a C-arm gantry, a base stand, and a bearing of the radiation therapy system of FIG. 1, as well as a direct-drive system for rotation of the C-arm gantry, according to various embodiments.
FIG. 6 schematically illustrates a second assembly of the drive system of FIG. 5, according to various embodiments.
FIG. 7 is a schematic plan view of a circular array of magnets that can be included in the second assembly of FIG. 6, according to various embodiments.
FIG. 8 schematically illustrates a first assembly of the drive system of FIG. 5, according to various embodiments.
FIG. 9 schematically illustrates the first assembly and the second assembly of the drive system of FIG. 5 positioned adjacent to each during operation, according to an embodiment.
FIG. 10 is a schematic plan view of a C-arm gantry, a drive system, and forces generated on the C-arm gantry by the drive system, according to various embodiments.
FIG. 11 schematically illustrates a more detailed view of a C-arm gantry, a base stand, and a bearing of the radiation therapy system of FIG. 1, as well as a direct-drive system for rotation of the C-arm gantry, according to various other embodiments.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here. It will be readily understood that the aspects of the disclosure, as generally described herein, and illustrated in the figures, can be arranged, substituted, combined, and designed in a wide variety of different configurations, all of which are explicitly contemplated and make part of this disclosure. Although the terms "first" and "second" are used to describe various elements, these elements should not be limited by these terms. These terms are used to distinguish one element from another. For example, a first element may be referred to as a second element, and vice versa. Independent of the grammatical term usage, individuals with male, female or other gender identities are included within the term.

### Introduction

As noted previously, radiation therapy systems commonly employ drive systems that enable precise and repeatable rotational positioning of a radiation source about a treatment couch. As requirements for radiation therapy systems become more stringent, such as higher gantry rotation speeds and lower tolerances for rotational error, the drawbacks of conventional drive systems become increasingly problematic. For example, gear-driven systems, such as spur, helical, or worm gear drives, are subject to gear backlash, which is incompatible with the high positional accuracy required for radiation therapy systems. Furthermore, such gear-driven systems require periodic lubrication, are generally impractical without the added space and complexity of large motors and/or gearboxes, and are susceptible to entanglement or damage by foreign objects. Similarly, belt drives and cable drives are also not suitable for high precision rotation applications due to slip and/or wear of the belt or cable, and require significant space to accommodate the size of the belts or cables needed to meet the performance requirements for rotating a gantry of a radiation therapy system. Further, as with gear-driven systems, belt drives and cable drives are susceptible to entanglement or damage by foreign objects. Roller chain systems are also susceptible to such hazards presented by foreign objects, and complicate precise repeatable rotation of a gantry caused by changes in chain tensioning and by elongation of the chain due to fatigue loading.

Accordingly, there is a need in the art for improved drive systems for rotationally positioning a radiation source of a radiation therapy system.

### System Overview

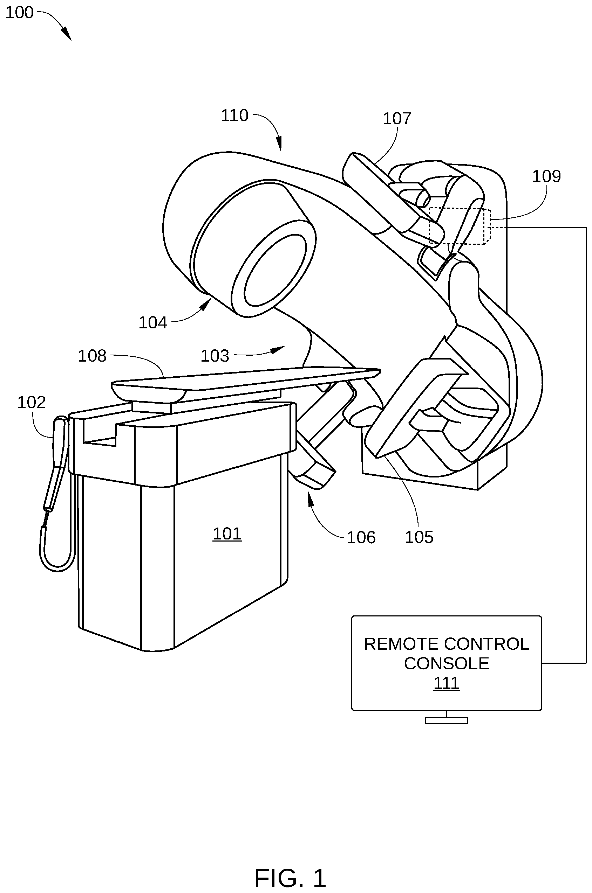

FIG. 1 is a perspective view of a radiation therapy system 100 that can beneficially implement various embodiments. Radiation therapy (RT) system 100 is a radiation system that may be configured to detect intra-fraction motion in near-real time using either optical or X-ray imaging techniques, or both. Thus, in some embodiments, RT system 100 is configured to provide stereotactic radiosurgery and precision radiotherapy for lesions, tumors, and conditions anywhere in the body where radiation treatment is indicated. As such, RT system 100 can include one or more of a linear accelerator (LINAC) 104 that generates an MV treatment beam of high energy X-rays or other radiation, one or more kilovolt (kV) imaging X-ray sources 106, one or more imaging panels 107 (e.g., an X-ray imager), and a mega-Volt (MV) electronic portal imaging device (EPID) 105. In the embodiment illustrated in FIG. 1, RT system 100 is configured with a C-arm gantry 110 capable of infinite rotation via a slip ring connection.

In some embodiments, RT system 100 is capable of X-ray imaging of a target volume immediately prior to and/or during application of an MV treatment beam, so that an image-guided radiation therapy (IGRT) and/or an intensity-modulated radiation therapy (IMRT) process can be performed using X-ray imaging. For example, in some embodiments, such processes can include kV imaging of the target volume in conjunction with imaging generated by the MV treatment beam. RT system 100 may include one or more touchscreens (not shown) for patient information verification, couch motion controls 102, a radiation area 103, a couch positioning assembly 101, a couch 108 disposed on couch positioning assembly 101, and an image acquisition and treatment control computer 109, all of which are disposed within a treatment room. RT system 100 further includes a remote control console 111, which is disposed outside the treatment room and enables treatment delivery and patient monitoring from a remote location. Couch positioning assembly 101 is configured to precisely position couch 108 with respect to radiation area 103. Motion controls 102 include input devices, such as buttons and/or switches, that enable a user to operate couch positioning assembly 101 to automatically and precisely position couch 108 to a predetermined location with respect to radiation area 103. Motion controls 102 also enable a user to manually position couch 108 to a particular location, such as a planned treatment position for a patient or anatomical target.

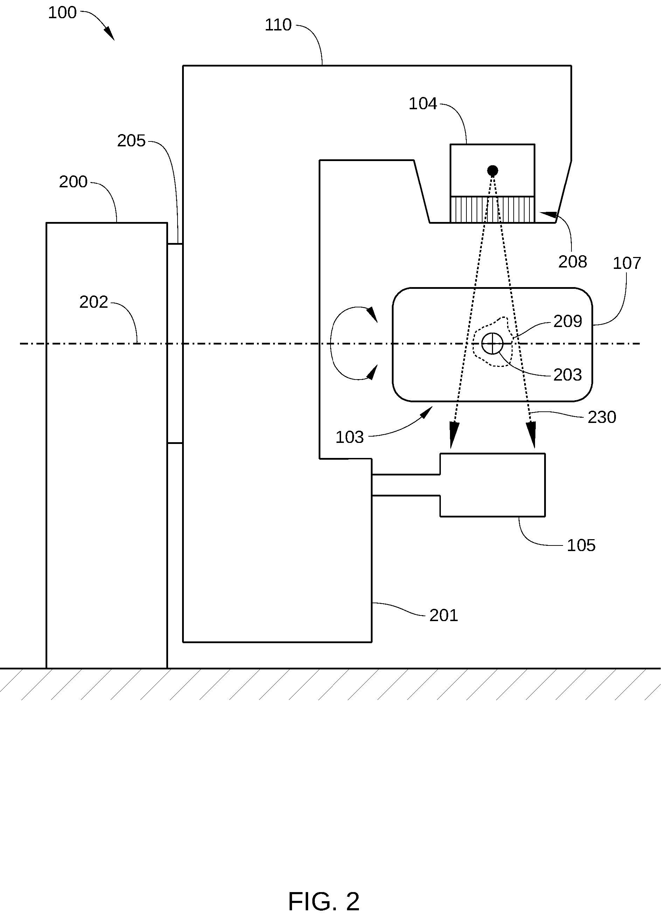

FIG. 2 schematically illustrates a side view of RT system 100, according to various embodiments. As shown, RT system 100 includes a base stand 200 and C-arm gantry 110. In FIG. 2, couch positioning assembly 101, couch 108, and imaging X-ray source 106 are omitted for clarity. Base stand 200 is a fixed support structure for components of RT treatment system 100, including C-arm gantry 110 and a drive system (not shown) for rotatably moving C-arm gantry 110 about a horizontal rotation axis 202. Base stand 200 rests on and/or is fixed to a support surface that is external to RT treatment system 100, such as a floor of an RT treatment facility. C-arm gantry 110 is rotationally coupled to base stand 200, for example via a bearing 205 (cross-hatched). C-arm gantry 110 is a support structure on which various components of RT system 100 are mounted, including LINAC 104, EPID 105, imaging X-ray source 106 (not shown in FIG. 2 for clarity), and imaging panel 107.

In the embodiment illustrated in FIG. 2, imaging panel 107 is depicted as a planar device, whereas in other embodiments, imaging panel 107 can have a curved configuration. In the embodiment illustrated in FIGS. 1 and 2, RT system 100 includes a single imaging panel and a single corresponding imaging radiation source in addition to EPID 105. In other embodiments, RT system 100 can include two or more imaging panels, each with a corresponding imaging radiation source. Further, in some embodiments, couch positioning assembly 101 is configured to rotate, pitch, roll, and/or translate couch 108 sequentially relative to isocenter 203 to one or more treatment positions.

LINAC 104 is a radiation source, and typically includes one or more of an electron gun for generating electrons, an accelerating waveguide, an electron beam target, an electron beam transport means (such as a bending magnet) for directing the electron beam to the electron beam target, and/or a collimator assembly 208 for collimating and shaping a treatment beam 230 that originates from the electron beam target. Collimator assembly 208 typically includes one or more of a primary collimator that defines the largest available circular radiation field for treatment beam 230, a secondary collimator for providing a rectangular or square radiation field at isocenter 203 (for example via X-jaws and Y-jaws), and a multileaf collimator (MLC) for conforming treatment beam 230 to a planning target volume (PTV) or other anatomical target. In other embodiments, LINAC 104 can be any other radiation source suitable for radiation therapy.

During radiation treatment, LINAC 104 is configured to generate treatment beam 230, which can include high-energy radiation (for example MV X-rays or MV electrons). In other embodiments, treatment beam 230 includes electrons, protons, and/or other heavy charged particles, ultra-high dose rate X-rays (e.g., for FLASH radiotherapy), and/or microbeams for microbeam radiation therapy. In addition, imaging panel 107 is configured to receive imaging radiation and generate suitable projection images therefrom. Further, in some embodiments, as treatment beam 230 is directed to isocenter 203 while C-arm gantry 110 rotates through a treatment arc, image acquisitions can be performed via EPID 105 to generate image data for target volume 209. For example, in such embodiments, EPID 105 generates one or more projection images of target volume 209 and/or a region of patient anatomy surrounding target volume 209. Thus, projection images (e.g., 2D X-ray images) of target volume 209 can be generated during portions of an IGRT or IMRT process via imaging panel 107 and/or EPID 105. Such projection images can then be employed to construct or update portions of imaging data for a digital volume that corresponds to a three-dimensional (3D) region that includes target volume 209. That is, a 3D image of such a 3D region is reconstructed from the projection images. In some embodiments, cone-beam computed tomography (CBCT) and/or digital tomosynthesis (DTS) can be used to process the projection images generated by imaging panel 107.

During operation of RT treatment system 100, C-arm gantry 110 rotates about radiation area 103 when actuated by the drive system for rotatably moving C-arm gantry 110 about horizontal rotation axis 202. Imaging X-ray source 106 is configured to direct a conical beam of X-rays, referred to herein as imaging X-rays (not shown in FIG. 2 for clarity), through an isocenter 203 of RT system 100 to imaging panel 107. Ideally, isocenter 203 corresponds to the location of a target volume 209 to be treated, such as a PTV, a gross tumor volume (GTV), a clinical target volume (CTV), and/or an internal target volume (ITV), among others. According to various embodiments, a drive system of RT treatment system 100 enables precise and repeatable rotational positioning of LINAC 104 (or any other suitable radiation source) about isocenter 203. Various embodiments of such a drive system are described below.

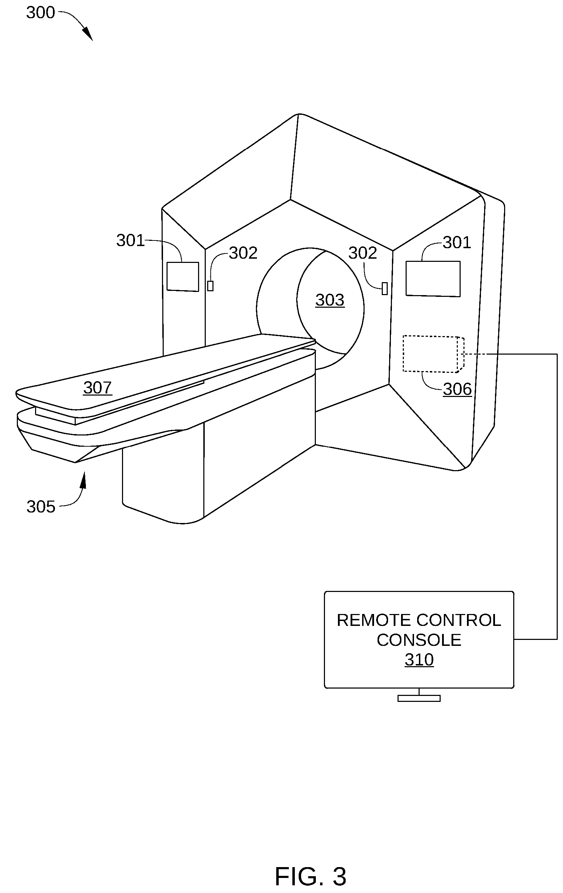

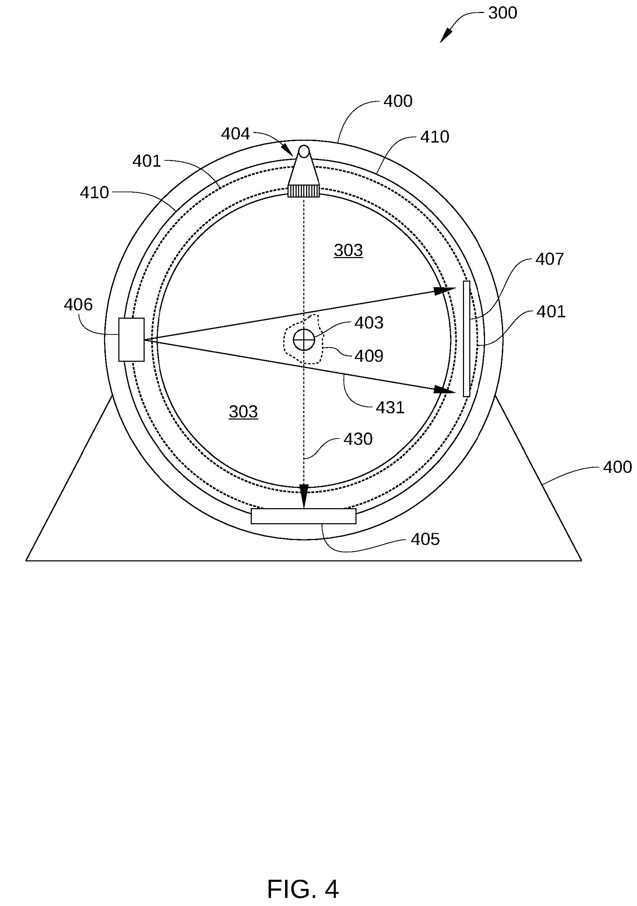

In the embodiment illustrated in FIGS. 1 and 2, C-arm gantry 110 of RT system 100 is capable of infinite rotation via a slip ring connection. In other embodiments, a radiation therapy system can be configured with a ring-based gantry that is disposed about an isocenter of the radiation therapy system. One such embodiment is described below in conjunction with FIGS. 3 and 4.

FIG. 3 is a perspective view of an RT system 300, according to various embodiments. In some embodiments, RT system 300 can be consistent with RT system 100 of FIGS. 1 and 2, except that RT system 300 is configured with a circular or ring-based gantry. As such, RT system 300 can include one or more touchscreens 301, couch motion controls 302, a bore 303, a base positioning assembly 305, a couch 307 disposed on base positioning assembly 305, and an image acquisition and treatment control computer 306, all of which are disposed within a treatment room. RT system 300 further includes a remote control console 310, which is disposed outside the treatment room and enables treatment delivery and patient monitoring from a remote location. Base positioning assembly 305 is configured to precisely position couch 307 with respect to bore 303, and motion controls 302 include input devices, such as button and/or switches, that enable a user to operate base positioning assembly 305 to automatically and precisely position couch 307 to a predetermined location with respect to bore 303. Motion controls 302 also enable a user to manually position couch 307 to a predetermined location.

FIG. 4 schematically illustrates a base stand 400 and gantry 410 of RT system 300, according to various embodiments. Covers, base positioning assembly 305, couch 307, and other components of RT system 300 are omitted in FIG. 4 for clarity. Base stand 400 is a fixed support structure for components of RT system 300, including gantry 410 and a drive system 401 (dashed lines) for rotatably moving gantry 410. Base stand 400 rests on and/or is fixed to a support surface that is external to RT system 300, such as a floor of a radiotherapy treatment facility. Gantry 410 is rotationally coupled to base stand 400 and is a support structure on which various components of RT system 300 are mounted, including a LINAC 404, an EPID 405, an imaging X-ray source 406, and an X-ray imager 407.

During operation of RT system 300, drive system 401 rotationally actuates gantry 410, so that gantry 410 rotates about bore 303. LINAC 404 generates an MV treatment beam 430 of high energy X-rays (or in some embodiments electrons, protons, and/or other heavy charged particles, ultra-high dose rate X-rays (e.g., for FLASH radiotherapy) or microbeams for microbeam radiation therapy) and EPID 405 is configured to acquire X-ray images with treatment beam 430. Imaging X-ray source 406 is configured to direct a conical beam of X-rays, referred to herein as imaging X-rays 431, through an isocenter 403 of RT system 300 to X-ray imager 407, and isocenter 403 typically corresponds to the location of a target volume 409 to be treated. In the embodiment illustrated in FIG. 4, X-ray imager 407 is depicted as a planar device, whereas in other embodiments, X-ray imager 407 can have a curved configuration. In the embodiment illustrated in FIG. 4, RT system 300 includes a single X-ray imager and a single corresponding imaging X-ray source. In other embodiments, RT system 300 can include two or more X-ray imagers, each with a corresponding imaging X-ray source.

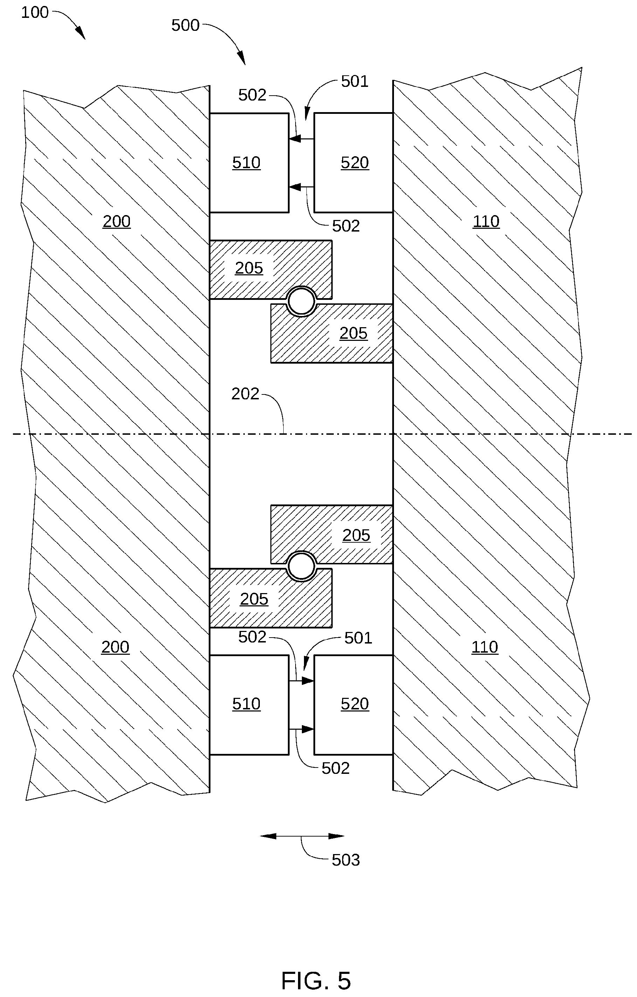

According to various embodiments, the drive system of RT system 100 and/or drive system 401 of RT system 300 includes a direct-drive electrical motor with a first assembly that is fixed to a base stand and a second assembly that is fixed to a rotatable gantry on which a treatment-delivering X-ray source is mounted. One embodiment of such a drive system is described below in conjunction with FIG. 5.

### Direct-Drive System for Gantry Rotation

FIG. 5 schematically illustrates a more detailed view of C-arm gantry 110, base stand 200, and bearing 205, as well as a drive system 500 for rotation of C-arm gantry 110, according to various embodiments. As shown, C-arm gantry 110 is mechanically coupled to base stand 200 via bearing 205, where bearing 205 enables C-arm gantry 110 to rotate about horizontal rotation axis 202. According to various embodiments, the rotation of C-arm gantry 110 about horizontal rotation axis 202 is enabled by drive system 500. While drive system 500 is described below with respect to RT system 100 and C-arm gantry 110, in other embodiments, drive system 500 can be implemented as drive system 401 of RT system 500.

Drive system 500 is configured as an axial flux electric motor that includes a first assembly 510 and a second assembly 520, where first assembly 510 is fixed to base stand 200 and second assembly 520 is fixed to C-arm gantry 110. As shown, first assembly 510 and second assembly 520 are separated from each other by an air gap 501, and therefore first assembly 510 and second assembly 520 are not mechanically coupled to each other. Drive system 500 is configured as a direct-drive system, in which there is no interposing drive train between first assembly 510 and second assembly 520.

In the embodiment illustrated in FIG. 5, magnetic flux 502 in an axial direction 503 (i.e., in a direction parallel to horizontal rotation axis 202) passes to and from components of first assembly 510 and from and to components of second assembly 520 via air gap 501. Further, magnetic flux (not shown in FIG. 5) also passes through components of first assembly 510 and second assembly 520 in a direction that is perpendicular to axial direction 503, which exerts torque about horizontal rotation axis 202 on the components of first assembly 510 and second assembly 520. Because first assembly 510 is fixed to base stand 200 and second assembly 520 is fixed to C-arm gantry 110, the torque exerted on the components of first assembly 510 and second assembly 520 about horizontal rotation axis 202 causes C-arm gantry 110 to rotate about horizontal rotation axis 202 via bearing 205. Thus, the electric motor of drive system 500 causes rotation of C-arm gantry 110 with no interposing drive train between first assembly 510 and second assembly 520. In some embodiments, a portion of bearing 205 that is fixed to C-arm gantry 110 also rotates about horizontal rotation axis 202 when the torque is exerted on components of first assembly 510 and second assembly 520.

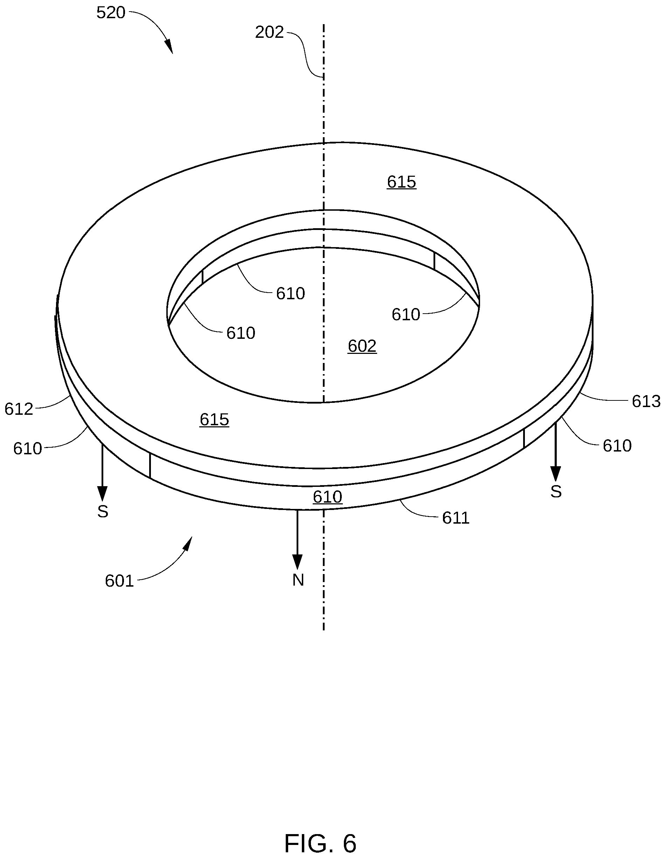

In some embodiments, first assembly 510 operates as a stator of the electric motor of drive system 500 and second assembly 520 operates as a rotor of the electric motor of drive system 500. In such embodiments, second assembly 520 includes an array of magnets coupled to C-arm gantry 110 and first assembly 510 includes a corresponding array of coils coupled to base stand 200. One such embodiment of drive system 500 is described below in conjunction with FIGS. 6 - 9.

### Direct-Drive System Using Axial Flux Motor

FIG. 6 schematically illustrates second assembly 520 of drive system 500, according to various embodiments. In the embodiment illustrated in FIG. 6, second assembly 520 includes a plurality of magnets 610 that are arranged in a circular array 601 around a center region 602 that is occupied by bearing 205 (shown in FIGS. 2 and 3). Thus, magnets 610 are arranged around a perimeter of bearing 205. Horizontal rotation axis 202 passes through center region 602 as shown during operation of drive system 500.

In some embodiments, magnets 610 are coupled to a magnet yoke 615, a plate, or some other structural component that enables magnets 610 to be fixed or coupled to C-arm gantry 110 (not shown for clarity) without contacting or interfering with bearing 205. In some embodiments, the material of magnet yoke 615 is selected to enhance magnetic flux between adjacent magnets during operation of drive system 500, such as a ferromagnetic material.

Generally, magnets 610 are arranged to have alternating polarity, so that each magnet 610 has an opposite polarity of the two magnets 610 adjacent thereto. For example, in the embodiment illustrated in FIG. 6, a first magnet 611 is positioned on magnet yoke 615 so that a north pole is directed downward as shown, while adjacent magnets 612 and 613 are positioned on magnet yoke 615 so that a south pole is directed downward as shown.

In some embodiments, magnets 610 are permanent magnets, and therefore do not require a flow of electrical current to generate a magnetic field. In such embodiments, magnets 610 can be permanent magnets that include a neodymium-based material, such as neodymium-iron-boron (NdFeB) magnets, or any other suitable material. In embodiments in which magnets 610 are permanent magnets, no electrical wiring is routed to magnets 610, which is advantageous when second assembly 520 is employed in the rotating portion of drive system 500, such as C-arm gantry 110. Alternatively, in some embodiments, magnets 610 can be electromagnets. In such embodiments, electrical power can be routed to magnets 610 via one or more slip-ring connections (not shown), which allow the transmission of power and/or electrical signals from a stationary structure (e.g., base stand 200 in FIGS. 2 and 3) to a rotating structure (e.g., C-arm gantry 110 in FIGS. 2 and 3).

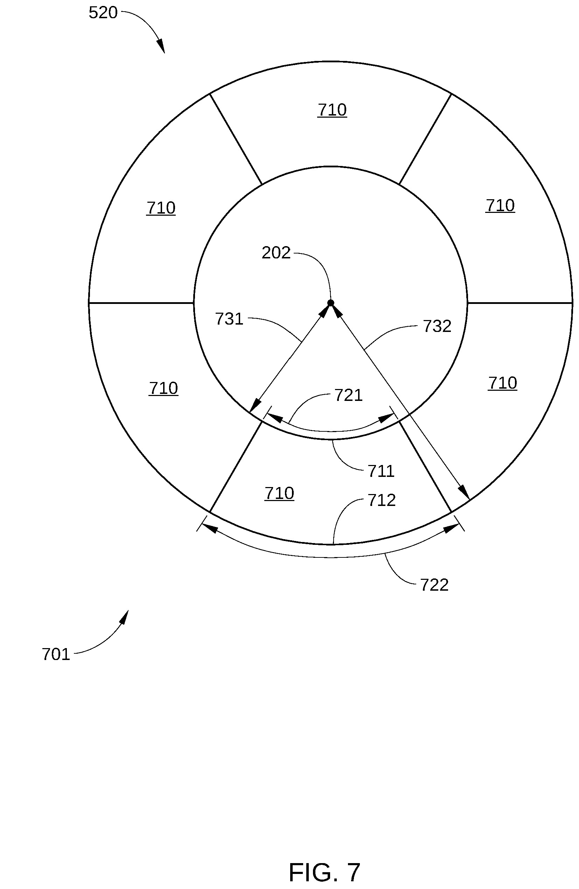

In some embodiments, magnets 610 are configured to produce as much magnetic flux (and thereby torque on C-arm gantry 110) as is practicable in the volume available for magnets 610 within C-gantry arm 110 or base stand 200. Thus, in some embodiments, magnets 610 are configured to substantially fill the volume available around bearing 205 in C-arm gantry 110 or in base stand 200. One such embodiment is described below in conjunction with FIG. 7.

FIG. 7 is a schematic plan view of a circular array 701 of magnets 710 that can be included in second assembly 520, according to various embodiments. As shown, magnets 710 are arranged around horizontal rotation axis 202, and each magnet 710 has a fan-shaped, tapered, or trapezoidal form factor. Thus, each magnet 710 has a first side 711 that has a first length 721 and is disposed at a first distance 731 from an axis of rotation of C-arm gantry 110 (such as horizontal rotation axis 202) and a second side 712 that has a second length 722 and is disposed at a second distance 732 from the axis of rotation of C-arm gantry 110, where first length 721 is less than second length 722 and first distance 731 is less than second distance 732. In addition, in some embodiments, each magnet 710 contacts each adjacent magnet 710, thereby eliminating dead space between magnets 710 and further increasing the magnetic flux produced by circular array 701.

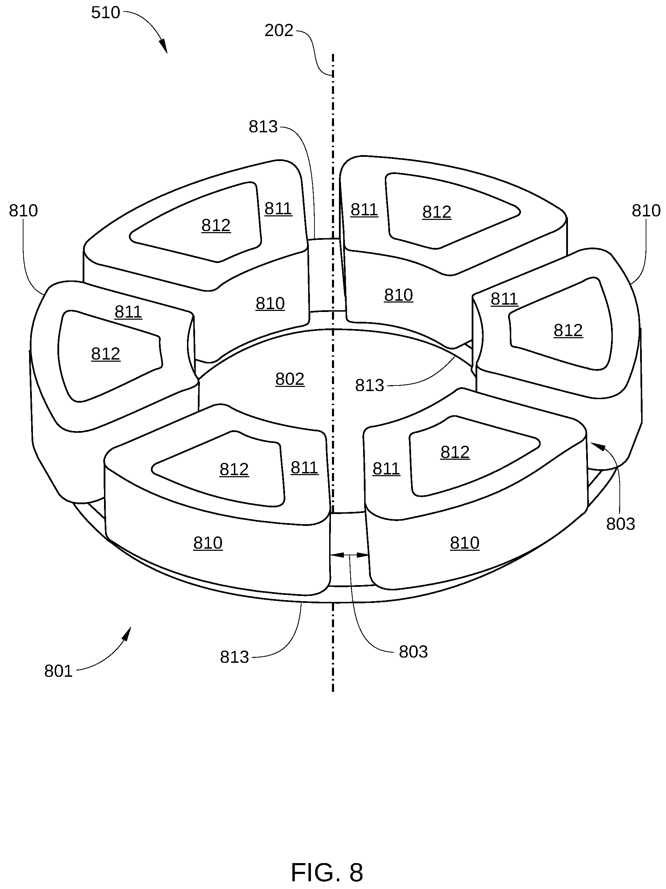

FIG. 8 schematically illustrates first assembly 510 of drive system 500, according to various embodiments. In the embodiment illustrated in FIG. 8, first assembly 510 is configured as a stator of drive system 500. As such, first assembly 510 includes a plurality of coils 810 that are arranged in a circular array 801 around a center region 802 that is occupied by bearing 205 (shown in FIGS. 2 and 3 and omitted for clarity in FIG. 8). Horizontal rotation axis 202 passes through center region 802 as shown during operation of drive system 500.

In some embodiments, each coil 810 includes a plurality of windings 811 that are wrapped around a respective core 812. For clarity, in FIG. 8 individual windings 811 are not depicted and are represented schematically as a monolithic layer around each core 812. Generally, cores 812 include a ferromagnetic material, such as steel or iron. In some embodiments, each core 812 is formed from a plurality of ferromagnetic plates, sometimes referred to "laminations," that have a lacquer coating. The laminations within cores 812 are not shown in FIG. 8 for clarity. In such embodiments, the laminations of a core 812 reduce eddy current flow within the core 812 as well as over-heating of the core 812. Thus, the use of laminations within each core 812 can reduce power loss and overheating. In radiation therapy, power efficiency and overheating of cores 812 is rarely a concern, due to the relatively low rotational speeds (e.g., 1 to 5 rpm) and the short time intervals during which rotation occurs. Thus, in some embodiments, each core 812 does not include laminations, and instead is formed from a solid block of ferromagnetic material, such as steel or iron.

In some embodiments, cores 812 are coupled together via a ferromagnetic plate 813. Ferromagnetic plate 813 facilitates magnetic flux between adjacent cores 812 during operation of drive system 500.

In the embodiment illustrated in FIG. 8, coils 810 can be similar in form factor to that of magnets 710 of FIG. 7, where coils 810 are arranged around horizontal rotation axis 202, and each coil 810 has a fan-shaped or trapezoidal form factor. In addition, in some embodiments, coils 810 are configured to produce as much magnetic flux as is practicable in the volume available for first assembly 510. Thus, in some embodiments, coils 810 are sized so that as small a gap 803 as practicable is present between adjacent coils 810. Further, in some embodiments, the number of coils 810 included in circular array 801 is the same as the number of magnets 710 included in circular array 701 of first assembly 510 (shown in FIG. 7).

In some embodiments, each coil 810 can be configured as a single-conductor coil. In such embodiments, the number of coils 810 can be selected to be a multiple of the number of phases of the drive (e.g., 3, 4, 5, etc). Alternatively, in some embodiments, each coil 810 can be a coil "pack" that includes three or more sub-coils. In such embodiments, the number of sub-coils in a coil pack can correspond to the number of phases of drive system 500 (e.g., 3, 4, 5, etc).

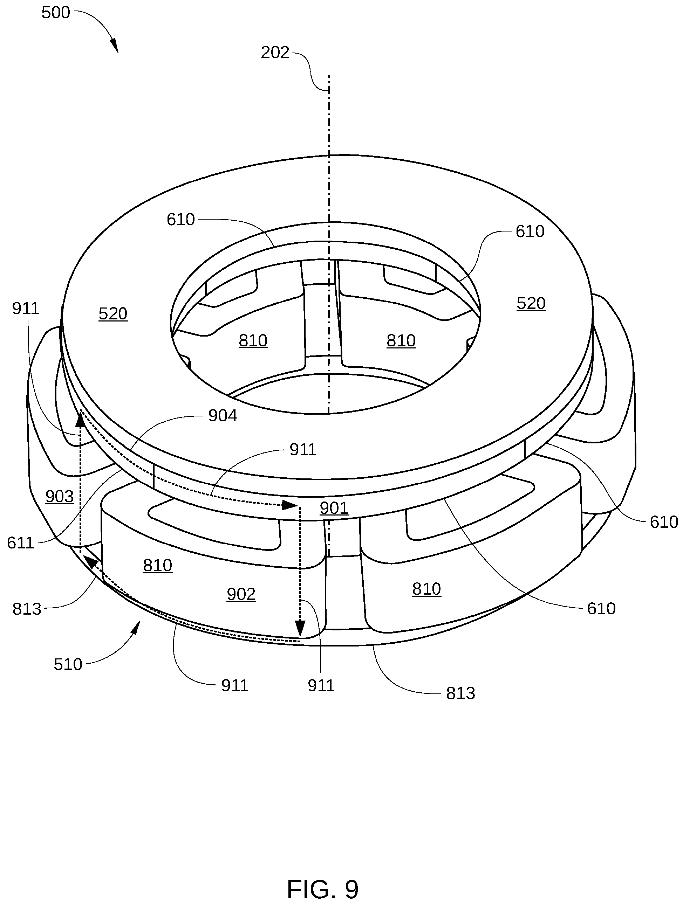

FIG. 9 schematically illustrates first assembly 510 and second assembly 520 positioned adjacent to each during operation, according to an embodiment. As shown, first assembly 510 includes a plurality of coils 810 in a planar array and second assembly 520 includes a plurality of magnets 610 in another planar array that is parallel to first assembly 510. Consequently, each magnet 610 overlaps, in a direction perpendicular to horizontal rotation axis 202, at least one coil 810. Similarly, each coil 810 overlaps at least one magnet 610 in a direction perpendicular to horizontal rotation axis 202. Thus, when drive system 500 causes C-arm gantry 110 to rotate, each coil 810 in first assembly 510 magnetically interacts with at least one magnet 610 of second array 520. Similarly, when drive system 500 causes C-arm gantry 110 to rotate, each magnet 610 in second assembly 520 magnetically interacts with at least one coil 810 of first array 510. As a result, when drive system 500 causes C-arm gantry 110 to rotate, magnetic flux exits a first magnet 901 in an axial direction parallel with horizontal rotation axis 202, passes through one or more coils 902, ferromagnetic plate 813, and one or more additional coils 903, enters a magnet 904 that is adjacent to first magnet 901 in the axial direction, then reenters first magnet 901, as shown by magnetic flux arrows 911 in FIG. 9.

In the embodiment illustrated in FIG. 9, the volume occupied by first assembly 510 and second assembly 520 is efficiently utilized, so that high magnetic flux is produced in a relatively small region. For example, besides the air gap between first assembly 510 and second assembly 520, the volume occupied by drive system 500 is almost entirely filled with magnets 610 and coils 810, and there is little dead space within drive system 500. Thus, in such embodiments, drive system 500 has a configuration of magnets and coils capable of providing high torque from a small volume. As noted previously, drive systems that include a mechanical drive train between a source of rotational motion and a C-arm gantry occupy significantly more volume than the embodiment of drive system 500 illustrated in FIG. 9, and therefore generally cannot produce as much torque per unit volume as drive system 500.

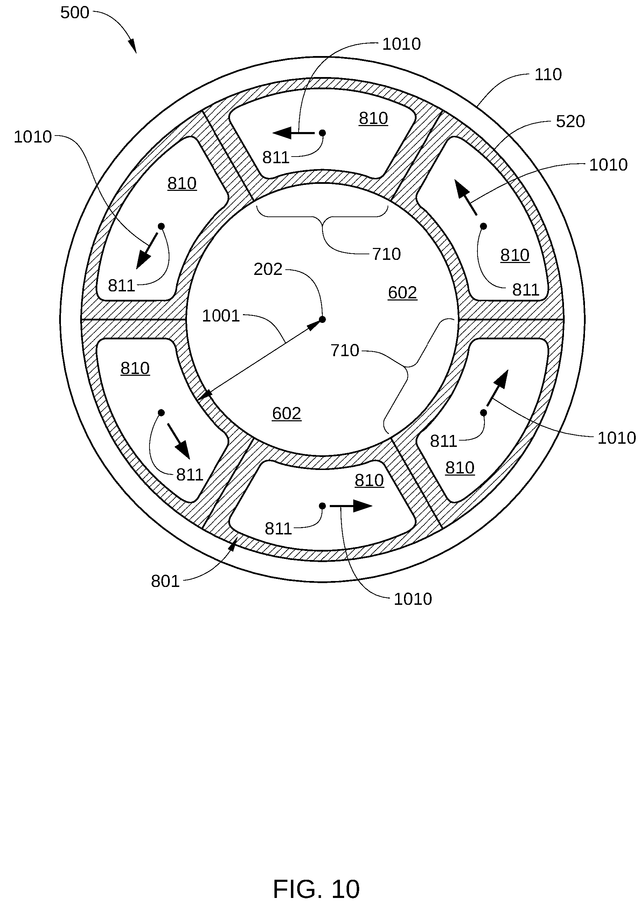

In some embodiments, coils 810 are arranged in a symmetric pattern about horizontal rotation axis 202, so that a net force acting on a gantry of a radiation therapy system is effectively zero when drive system 500 causes C-arm gantry 110 to rotate. Having net zero force on the gantry beneficially eliminates strain that is caused by a non-zero force exerted on the gantry. Such non-zero force can disturb the geometric accuracy of diagnostics and treatment being performed by the radiation therapy system. One such embodiment is described below in conjunction with FIG. 10.

FIG. 10 is a schematic plan view of C-arm gantry 110, drive system 500, and forces generated on C-arm gantry 110 by drive system 500, according to various embodiments. As shown, drive system 500 includes magnets 710 (cross-hatched) and circular array 801 of coils 810. As described previously, in some embodiments, magnets 710 can be included in second assembly 520 (also shown in FIG. 5), which is configured as a rotor of drive system 500 and is mounted on or coupled to C-arm gantry 110. In such embodiments, coils 810 can be included in first assembly 510 (shown in FIG. 5), which is configured as a stator of drive system 500 and is mounted on or coupled to a base stand of RT system 100. In operation, each of coils 810 exerts a rotational force 1010 on C-arm gantry 110. Because coils 810 are symmetrically positioned about horizontal rotation axis 202, the net force exerted on C-arm gantry 110 is zero. By contrast, the net torque applied to C-arm gantry 110 by coils 810 is a function of each rotational force 1010, and therefore is greater than zero.

According to various embodiments, any number of coils 810 greater than one can be symmetrically arranged to produce zero net force on C-arm gantry 110 when in operation. In the embodiment illustrated in FIG. 10, a group of six coils 810 are symmetrically positioned around a circumference of, for example, center region 602 of drive system 500. In such an embodiment, coils 810 are positioned at an equal radial distance 1001 from horizontal rotation axis 202 and symmetrically distributed circumferentially about horizontal rotation axis 202. Thus, in the embodiment illustrated in FIG. 10, a center point 811 of each coil 810 is circumferentially separated from each adjacent coil 810 by 60 degrees. By contrast, in an embodiment in which drive system 500 includes two coils 810, each coil 810 is circumferentially separated from each other coil 810 by 180 degrees, in an embodiment in which drive system 500 includes three coils 810, each coil 810 is circumferentially separated from each other coil 810 by 120 degrees, in an embodiment in which drive system 500 includes four coils 810, each coil 810 is circumferentially separated from each other coil 810 by 90 degrees, and so on.

In the embodiments described above in conjunction with FIGS. 7 - 10, drive system 500 includes an axial flux motor with a single stator and a single rotor for causing rotation of C-arm gantry 110. Alternatively, in other embodiments, a drive system for rotating a radiation therapy gantry includes a single-stator, dual-rotor axial flux motor. For example, in such embodiments, the drive system includes a first assembly coupled to a base stand of a radiation therapy system and a second assembly coupled to a rotatable gantry of the radiation therapy system. In such embodiments, the second assembly may include two rotors of an axial flux motor that are each coupled to a single shaft that rotates the rotatable gantry. Alternatively, in yet other embodiments, a drive system for rotating a radiation therapy gantry includes a dual-stator, single rotor axial flux motor. For example, in such embodiments, the drive system includes a first assembly coupled to a base stand of a radiation therapy system and a second assembly coupled to a rotatable gantry of the radiation therapy system, where the first assembly may include two stators of an axial flux motor and the second assembly includes a single rotor that is coupled to a shaft that rotates the rotatable gantry.

### Direct-Drive System Using Radial Flux Motor

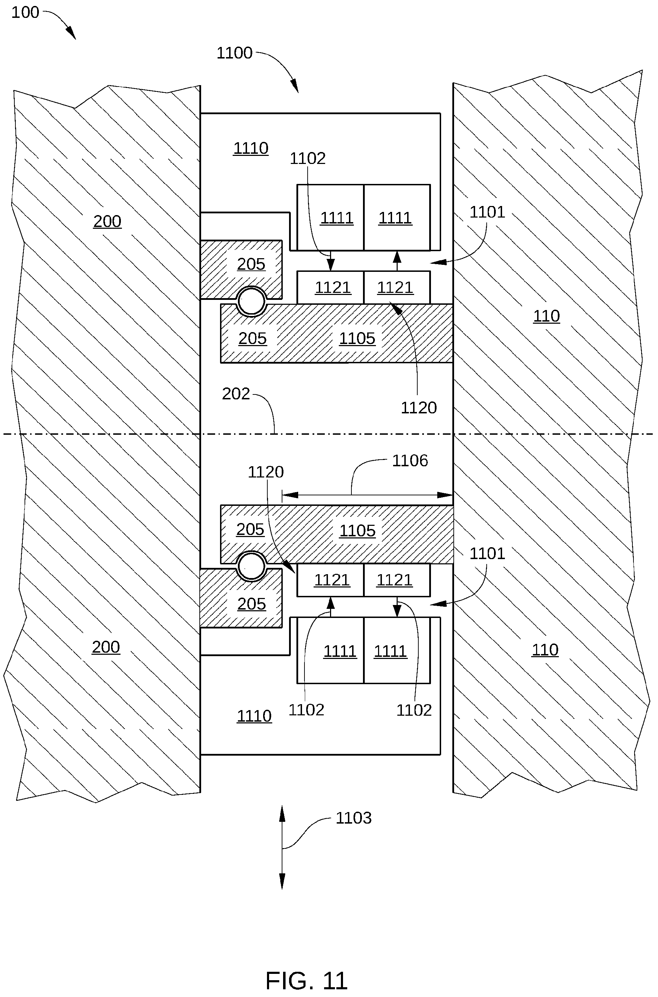

In the embodiments described above in conjunction with FIGS. 7 - 10, drive system 500 for rotation of C-arm gantry 110 is implemented via an axial flux motor. In other embodiments, drive system 500 is implemented via a radial flux motor. In such embodiments, magnetic flux is generated by the radial flux motor in a radial direction, i.e., perpendicular to the axis of rotation of the radial flux motor and C-arm gantry 110. One such embodiment is described below in conjunction with FIG. 11.

FIG. 11 schematically illustrates a detailed view of C-arm gantry 110, base stand 200, and bearing 205, as well as a direct-drive system 1100 for rotation of C-arm gantry 110, according to various embodiments. As shown, C-arm gantry 110 is mechanically coupled to base stand 200 via bearing 205, where bearing 205 enables C-arm gantry 110 to rotate about horizontal rotation axis 202. In the embodiment illustrated in FIG. 11, bearing 205 is coupled to C-arm gantry 110 via a shaft 1105. According to various embodiments, the rotation of C-arm gantry 110 about horizontal rotation axis 202 is enabled by direct-drive system 1100.

In the embodiment illustrated in FIG. 11, direct-drive system 1100 is configured as a radial flux motor that includes a first assembly 1110 and a second assembly 1120, where first assembly 1110 is fixed to base stand 200 and second assembly 1120 is fixed to C-arm gantry 110. In other embodiments, direct-drive system 1100 can be implemented in a ring-gantry-based radiation therapy system, such as RT system 300 in FIGS. 3 and 4.

As shown, first assembly 1110 and second assembly 1120 are separated from each other by an air gap 1101, and therefore first assembly 1110 and second assembly 1120 are not mechanically coupled to each other. In the embodiment illustrated in FIG. 11, magnetic flux 1102 in a radial direction 1103 (i.e., in a direction perpendicular to horizontal rotation axis 202) passes between components of first assembly 1110 and second assembly 1120 via air gap 1101. For example, in FIG. 11, magnetic flux 1102 passes radially from coils 1111 of first assembly 1110 to and from magnets 1121 of second assembly 1120. Further, magnetic flux (not shown in FIG. 11) also passes through components of first assembly 1110 and second assembly 1120 in a direction that is perpendicular to axial direction 1103, which exerts torque about horizontal rotation axis 202 on the components of first assembly 1110 and second assembly 1120. Because first assembly 1110 is fixed to base stand 200 and second assembly 1120 is fixed to C-arm gantry 110, the torque exerted on the components of first assembly 1110 and second assembly 1120 about horizontal rotation axis 202 causes C-arm gantry 110 to rotate about horizontal rotation axis 202 via bearing 205. Thus, the electric motor of direct-drive system 1100 causes rotation of C-arm gantry 110 with no interposing drive train between first assembly 1110 and second assembly 1120.

As shown, first assembly 1110 includes a plurality of coils 1111 in an array that is distributed radially about shaft 1105 and second assembly 520 includes a plurality of magnets 1121 in array distributed on a surface of shaft 1105. In the embodiment illustrated in FIG. 11, coils 1111 and magnets 1121 are aligned with each other along a direction parallel with horizontal rotation axis 202. Alternatively, in some embodiments, coils 1111 and magnets 1121 can be offset from each other in the direction parallel with horizontal rotation axis 202. Further, in the embodiment illustrated in FIG. 11, each magnet 1121 overlaps, in a circumferential direction (e.g., out of the page in FIG. 11) at least one coil 1111. Similarly, each coil 1111 overlaps at least one magnet 1121 in the circumferential direction perpendicular to horizontal rotation axis 202. Thus, when drive system 1100 causes C-arm gantry 110 to rotate, each coil 1111 in first assembly 1110 magnetically interacts with at least one magnet 1121 of second array 1120. Similarly, when drive system 1100 causes C-arm gantry 110 to rotate, each magnet 1121 in second assembly 1120 magnetically interacts with at least one coil 1111 of first array 1110. As a result, when drive system 1100 causes C-arm gantry 110 to rotate, magnetic flux passes between magnets 1121 and coils 1111 in a radial direction 1103 that is perpendicular to horizontal rotation axis 202.

In the embodiment illustrated in FIG. 11, first assembly 1110 is coupled to base stand 200, and second assembly 1120 is coupled to C-arm gantry 110, for example via shaft 1105. In such embodiments, power does not need to be routed to second assembly 1120 when magnets 1121 are permanent magnets. Alternatively, in some embodiments, first assembly 1110 can be fixed to C-arm gantry 110 and second assembly 1120 can be fixed to base stand 200. In such embodiments, electrical power can be routed to magnets 1121 via one or more slip-ring connections (not shown), which allow the transmission of power and/or electrical signals from a stationary structure (e.g., base stand 200) to a rotating structure (e.g., C-arm gantry 110).

It is noted that the number of coils 1111 that can be included in first assembly 1110 and the number of magnets 1121 that can be included in second assembly 1120 is limited by a length 1106 of shaft 1105. Thus, for drive system 500 to generate higher torque via a radial flux motor as depicted in FIG. 11, length 1106 of shaft 1105 can be increased to accommodate more coils 1111 in first assembly 1110 and more magnets 1121 in second assembly 1120.

### Direct-Drive System Using Transverse Flux Motor

In the embodiments described above in conjunction with FIG. 11, drive system 500 for rotation of C-arm gantry 110 is implemented via a radial flux motor. In other embodiments, drive system 500 is implemented via a transverse flux motor. In such embodiments, magnetic flux can be generated by the transverse flux motor in either an axial direction or a radial direction. This is because transverse flux motors can be configured with either an axial air gap, as described above in conjunction with FIG. 5, or a radial air gap, as described above in conjunction with FIG. 11. Thus, in some embodiments, drive system 500 for rotation of C-arm gantry 110 can include a transverse flux motor that has a disk-shaped rotor (as shown in FIG. 5) or a cylindrical rotor (as shown in FIG. 11.). When drive system 500 includes a transverse flux motor that has such a disk-shaped rotor, magnetic flux generated by the transverse flux motor passes across an air gap in an axial direction. By contrast, when drive system 500 includes a transverse flux motor that has such a cylindrical rotor, magnetic flux generated by the transverse flux motor passes across an air gap in a radial direction.

In a transverse flux motor, the winding approach for the stator differs from radial flux motors and axial flux motors. Instead of having stator teeth or poles with copper wire wound around them, a transverse flux motor has coils circumferentially around the axis of rotation. This approach enables the 3D flow of magnetic flux, crossing axially through the stator, circumferentially through the rotor, and radially through the air gap between the stator and the rotor.

While various aspects and embodiments have been disclosed herein, other aspects and embodiments will be apparent to those skilled in the art without departing from the scope and spirit of the described embodiments. The various aspects and embodiments disclosed herein are for purposes of illustration and are not intended to be limiting, with the true scope and spirit being indicated by the following claims.

## Claims

1. A radiation treatment system comprising:
a base stand configured to be fixed to or rest on a support surface external to the radiation treatment system;
a rotatable gantry with a treatment-delivering radiation source mounted thereon, wherein the rotatable gantry is rotatably coupled to the base stand via a bearing; and
an electric motor for rotating the rotatable gantry and a portion of the bearing fixed to the gantry, wherein the electric motor includes:
a first assembly that is fixed to the base stand; and
a second assembly that is fixed to the gantry and is separated from the first assembly by an air gap,
wherein magnetic flux across the air gap causes the electric motor to rotate the rotatable gantry and the portion of the bearing fixed to the rotatable gantry.

2. The radiation treatment system of claim 1, wherein:
the first assembly comprises a rotor of the electric motor and the second assembly comprises a stator of the electric motor; or
the first assembly comprises a stator of the electric motor and the second assembly comprises a rotor of the electric motor.

3. The radiation treatment system of claim 1 or 2, wherein the electric motor comprises one of an axial flux motor, a radial flux motor, or a transverse flux motor.

4. The radiation treatment system of claim 3, wherein magnetic flux generated by the transverse flux motor passes across the air gap in an axial direction.

5. The radiation treatment system of any one of claims 1 to 4, wherein the first assembly and the second assembly comprise an axial flux motor that includes a circular array of magnets arranged around a perimeter of the portion of the bearing.

6. The radiation treatment system of claim 5, wherein the second assembly comprises a rotor of the axial flux motor and includes the circular array of magnets.

7. The radiation treatment system of claim 5 or 6, wherein the first assembly comprises a stator of the axial flux motor.

8. The radiation treatment system of claim 7, wherein stator of the axial flux motor includes an array of coils, and wherein, optionally, each coil in the array of coils comprises a solid core.

9. The radiation treatment system of any one of claims 5 to 8, wherein each magnet in the circular array of magnets has:
a first side that has a first length and is disposed at a first distance from an axis of rotation of the gantry; and
a second side that has a second length and is disposed at a second distance from the axis of rotation of the gantry,
wherein the first length is less than the second length and the first distance is less than the second distance.

10. The radiation treatment system of any one of claims 5 to 9, wherein:
the axial flux motor comprises a single stator and a single rotor; and/or
each magnet in the circular array of magnets comprises a permanent magnet.

11. The radiation treatment system of any one of claims 5 to 10, wherein:
each magnet in the circular array of magnets magnetically interacts with at least one coil of a rotor of the axial flux motor when the electrical motor causes the rotatable gantry to rotate; or
each coil of a rotor of the axial flux motor magnetically interacts with at least one magnet in the circular array of magnets when the electrical motor causes the rotatable gantry to rotate.

12. The radiation treatment system of any one of claims 1 to 11, wherein there is no interposing mechanical drivetrain between the first assembly and the second assembly.

13. The radiation treatment system of any one of claims 1 to 12, wherein the second assembly comprises a rotor of an axial flux motor and includes an array of coils that are arranged in a symmetric pattern about an axis of rotation of the gantry.

14. The radiation treatment system of claim 13, wherein a net force acting on the gantry by the array of coils is zero when the axial flux motor causes the gantry to rotate.

15. The radiation treatment system of any one of claims 1 to 14, wherein:
the first assembly includes an array of coils,
the second assembly includes an array of magnets, and
each magnet in the array of magnets overlaps at least one coil in the array of coils;
or:
the first assembly includes an array of magnets,
the second assembly includes an array of coils, and
each magnet in the array of magnets overlaps at least one coil in the array of coils.
